(19) Europäisches Patentamt
European Patent Office
Office européen des brevets

(11) **EP 1 816 188 A1**

(12) **EUROPEAN PATENT APPLICATION**

(43) Date of publication:
**08.08.2007 Bulletin 2007/32**

(51) Int Cl.:
***C12M 1/36*** (2006.01)

(21) Application number: **06018900.8**

(22) Date of filing: **08.09.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **03.02.2006 JP 2006026484**

(71) Applicant: **Hitachi Plant Technologies, Ltd.**
**Chiyoda-ku**
**Tokyo 101-0047 (JP)**

(72) Inventor: **Amano, Ken**
**Hitachi, Ltd.**
**IPG**
**12th Floor**
**Chiyoda-ku**
**Tokyo 100-8220 (JP)**

(74) Representative: **Strehl Schübel-Hopf & Partner**
**Maximilianstrasse 54**
**80538 München (DE)**

(54) **Operation controller of culture tank**

(57)    Provided is an operation controller of a culture tank which can more precisely control the internal state of the culture tank by specifying a relatively small number of internal state variables, thus constructing a mathematical model which describes intracellular conditions by using these internal state variables, and thereby incorporating observable external variables thereto. The operation controller includes: a culture tank in which a culture medium for culturing animal cells or microorganism is enclosed, a measuring device for measuring concentrations of nutrient components, concentrations of products, and the number density of cells in the culture medium; a supply device for replenishing the nutrient components and oxygen into the culture medium; and an arithmetic processing unit in which the measured values from the measuring device are inputted, and which thus controls the supply device. The arithmetic processing unit solves an equation derived from a circuit network of intracellular reaction rates stored in a storage device by using temporal rates of change of the concentrations of the nutrient components, of the concentrations of the products, and of the number density of the cells as input data, and thus calculates a desired intracellular reaction rate. Accordingly, the arithmetic processing unit controls the supply device based on the results of the calculation, whereby the arithmetic processing unit controls the concentrations of the components contained in the culture medium.

FIG. 1

**Description**

**BACKGROUND OF THE INVENTION**

Field of the Invention

The present invention relates to an operation controller of a culture tank in which animal cells or microorganism are cultured to harvest useful products.

Description of the Related Art

The animal cells or microorganism are industrially cultured by stirring a culture solution while supplying nutrient components such as sugar and amino acid, and oxygen into a culture medium to cause the cells to grow. Thus, useful materials produced by the cells are harvested.

For example, Patent Document 1 describes a method of controlling the culture of the animal cells. That is, in the process of culturing the animal cells, at the first stage, the total amount of gas to be supplied to the culture tank is determined by means of a fuzzy inference from the measured values of the density and activity level of the cell being cultured. At the second stage, a gas composition is determined by means of the fuzzy inference from the measured values of the density of the dissolved oxygen and PH of the culture solution. The amount of the gas of each component to be supplied is determined from the value determined at the first and second stages to supply the gas to the culture tank.

Patent Document 2 describes a nonsteady and nonlinear phenomenon related to the number concentration of the microorganism, the substrate concentration, and the concentration of the dissolved oxygen in the water system containing the substrate and the microorganism using a differential equation. This differential equation is converted into a difference equation. The numerical solution of the differential equation using the parameters used in this difference equation as variables is determined. The degradation ability of the microorganism for the substrate is measured. Then, a comparison is made between the numerical solution of the difference equation and the measured value of the degradation ability of the microorganism for the substrate. A method of predicting a purification reaction in which the numerical solution closest to the measured value is specified as the behavior of the number concentration of the microorganism, the substrate concentration, and the concentration of the dissolved oxygen in the water tank is described in Patent Document 2.

Patent Document 3 describes a yeast culture method in which a target value of the quantitative value of a fermentation performance is previously specified in the production of yeast to control, based on the specified target value, various kinds of operations such as the supply of nutrient sources which control the growth rate of the yeast to culture, the adjustment of temperature and the like using a mathematical model.

Patent Document 1: Japanese Patent Application Laid-open Official Gazette No. Hei 5-103663

Patent Document 2: Japanese Patent Application Laid-open Official Gazette No. 2002-95496

Patent Document 3: Japanese Patent Application Laid-open Official Gazette No. Hei 9-65873

It is necessary for culture to suitably adjust internal conditions of the tank for the growth of the cells and the formation of products. In the technologies disclosed in Patent Documents 1, 2, and 3, the measurable conditions such as the concentrations of the nutrient components and oxygen in the culture medium, the number of revolutions of stirring, the density of the cells, the number concentration of the microorganism, the concentration of the dissolved oxygen and the like are measured to control the conditions. However, the substantial material production process is controlled by the metabolic reaction in the cells. Then, the internal conditions of the cells cannot directly be measured for control. That is, it is on a "black box" basis.

Therefore, only the measured information out of the cells including the concentrations of the nutrient components, the concentrations of the products, and the number density of the cells often causes the circumstances where a control cannot suitably be performed. This is true of the conventional technologies in which an experience-based operation, or a rule-based control under which a computer is caused to store past experiences as a rule to estimate a phenomenon corresponding to observed data, and to apply a control rule, is performed.

It is a first object of the present invention to provide the operational controller of the culture tank which can precisely control the internal conditions of a culture tank in combination with observable external variables by selecting a relatively small number of intracellular condition variables to construct a mathematical model which describes the intracellular conditions using the selected parameters.

It is a second object of the present invention to provide the operational controller of a culture tank which includes a calculation algorithm for finding out suitable operational conditions of the culture tank by using a value vector including the groups of external observation variables, such as the concentrations of the nutrient components, the concentrations of the products, and the number density of the cells, and the above intracellular condition variables as the internal condition parameters of the culture tank.

# EP 1 816 188 A1

## SUMMARY OF THE INVENTION

In order to achieve the above objects, the operation controller of the culture tank of the present invention includes a culture tank in which a culture medium for culturing animal cells or microorganism is enclosed, a measuring device for measuring the concentrations of the nutrient components, the concentrations of the products, and the number density of the cells in the culture medium, a supply device for replenishing the nutrient components and oxygen into the culture medium, and an arithmetic processing unit in which the measured values from the measuring device are inputted, and which controls the supply device. The arithmetic processing unit solves an equation derived from a circuit network of intracellular reaction rates stored in a storage device by using temporal rates of change of the concentrations of the nutrient components, of the concentrations of the products, and of the number density of the cells as input data, such that the arithmetic processing unit calculates a desired intracellular reaction rate. The arithmetic processing unit thus controls the supply device depending on the result of the calculation to control the concentrations of the components contained in the culture medium.

Moreover, the operation controller of the culture tank of the present invention includes a culture tank in which a culture medium for culturing animal cells or microorganism is enclosed, a measuring device for measuring the concentrations of nutrient components, the concentrations of the products, and the number density of the cells in the culture medium, a supply device for replenishing the nutrient components, and oxygen into the culture medium, and an arithmetic processing unit in which the measured values from the measuring device are inputted, and which thus controls the supply device. The arithmetic processing unit specifies target conditions such as a formation reaction rate and the like. The arithmetic processing unit solves an equation derived from a circuit network of intracellular reaction rates stored in a storage device by using temporal rates of change of the concentrations of the nutrient components, of the concentrations of the products, and of the number density of the cells as input data, such that the arithmetic processing unit calculates a set of intracellular reaction rates which meets the target conditions, and The arithmetic processing unit thus controls the supply device depending on the result of the calculation to control the concentrations of the components contained in the culture medium.

According to the present invention, the operation of the culture tank can suitably be controlled using the observation variables in the culture medium, such as the concentrations of nutrient components, the concentrations of the products, and the number density of the cells and the like, and the intracellular reaction rate as the condition variable of the culture tank.

## BRIEF DESCRIPTION OF THE DRAWINGS

Fig. 1 is a block diagram of an operation controller of a culture tank which is an example of the present invention.
Fig. 2 is a plan view showing an example of a display screen of an output display device of the present embodiment.
Fig. 3 is a plan view showing an example of a display screen of the output display device of the present embodiment.
Fig. 4 shows time integration algorithm of a model estimation equation.

## DETAILED DESCRIPTION OF THE PREFERRED EMBODIMENT

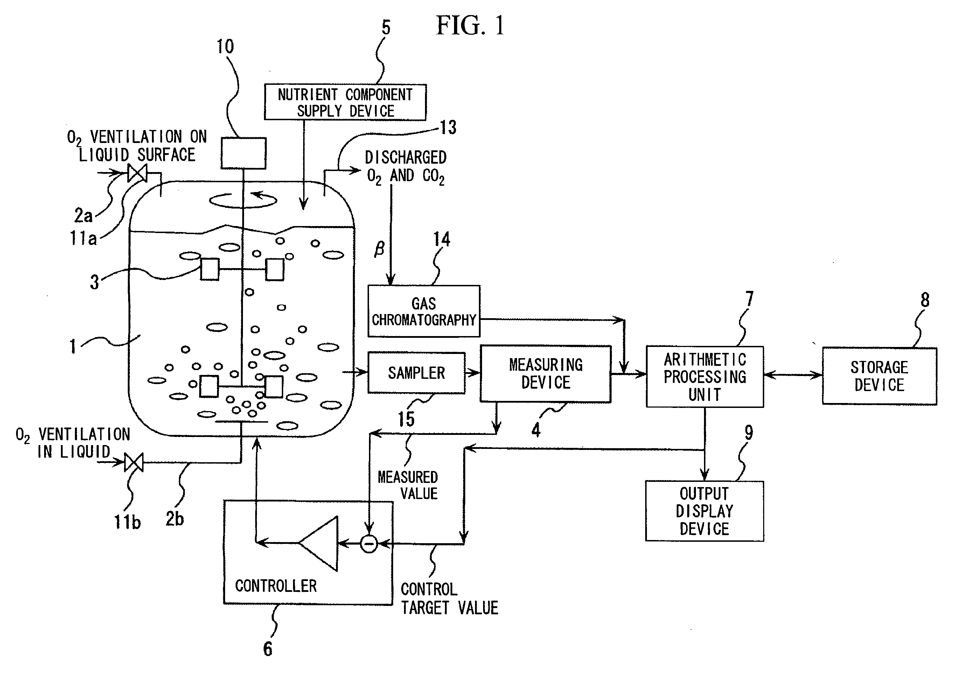

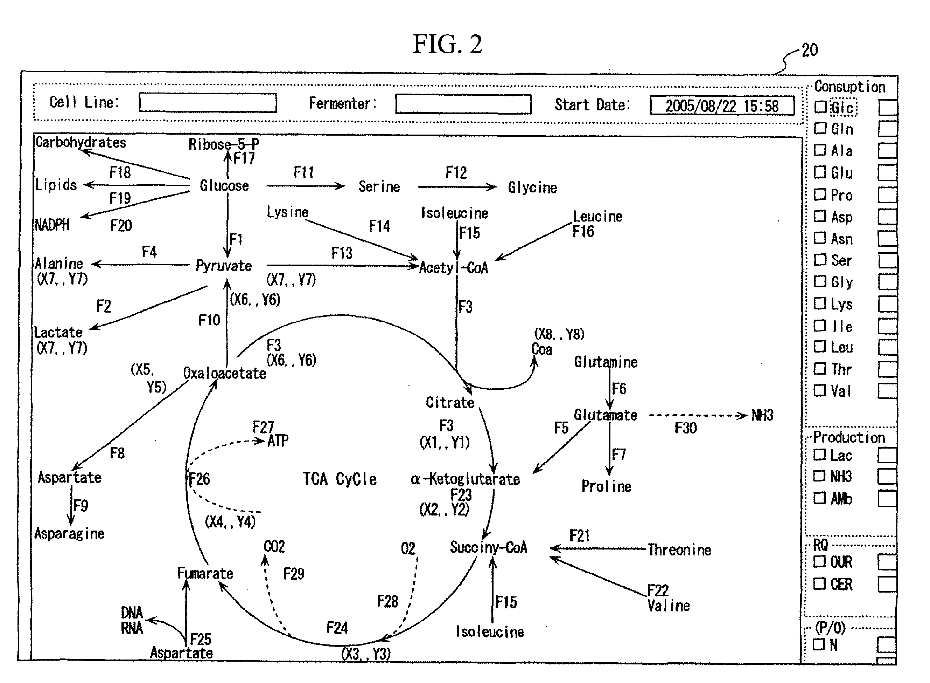

An example of the present invention will be described with reference to Figs. 1 to 4. Fig. 1 is a block diagram of the operation controller of a culture tank according to the present embodiment.

In a culture tank 1, a stirring blade 3 having blades at higher and lower positions in the tank is mounted. The stirring blade 3 is rotatably driven by a driving device 10 mounted above the culture tank 1. The culture tank 1 is provided thereabove with an air pipe 2a having a valve 11a, and therebelow with an air pipe 2b having a valve 11b. The air pipe 2a is used to vent oxygen gas therethrough to the direction of a liquid surface. The air pipe 2b is used to vent oxygen gas therethrough into the liquid. A waste gas pipe 13 is mounted above the culture tank 1, and is used to discharge oxygen gas and carbon dioxide gas. The culture tank 1 encloses therein the culture medium for culturing the cells, and animal cells or microorganism, and is provided with a nutrient component supply device 5 for supplying nutrient components such as sugar and amino acid with the culture medium.

A gas chromatograph 14 is connected to the waste gas pipe 13, which analyzes the oxygen gas and the carbon dioxide discharged from the waste gas pipe 13. The culture tank 1 is connected to a sampler 15 for detecting the state of the culture medium, which samples part of the culture medium to detect the state of the culture medium by means of a measuring device 4 connected to the sampler 15. The measuring device 4 measures the concentrations of the nutrient components such as sugar typified by glucose and amino acids typified by glutamine, the concentrations of products such as the concentration of lactic acid, the concentration of ammonia, and the concentration of protein, oxygen concentration, and the number density of the cells.

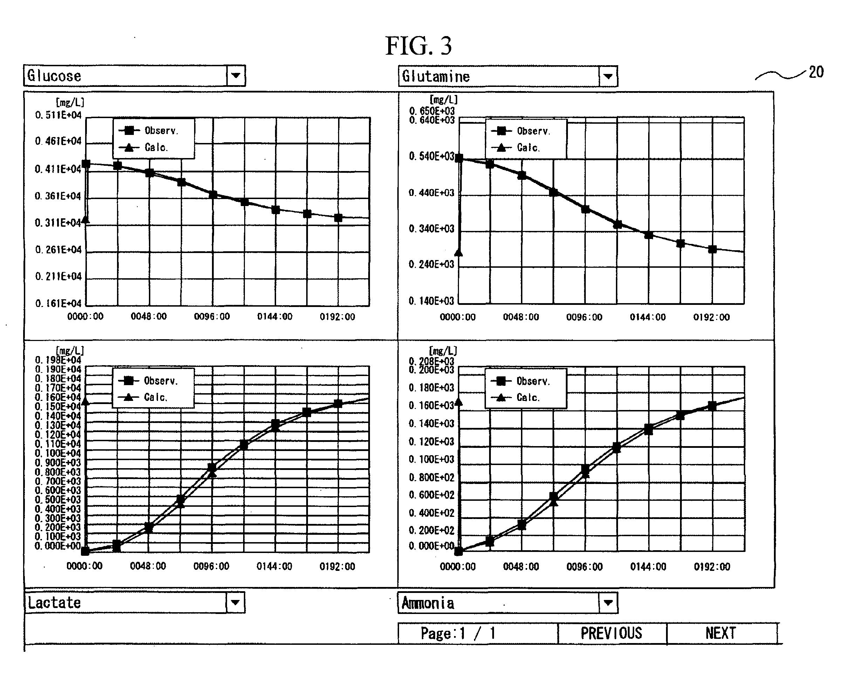

The gas chromatograph 14 and the measuring device 4 are connected to an arithmetic processing unit 7. The arithmetic processing unit 7 is connected to a storage device 8 and an output display device 9. A controller 6 is connected to the measuring device 4 which feeds back the measured value to the controller 6. The target control value calculated by the arithmetic processing unit 7 is inputted to the controller 6. The controller 6 outputs a PID control signal to the driving device 10, the valves 11 a and 11b, the nutrient component supply device 5 based on the measured value which has been fed back by the measuring device 4. The output display device 9 displays, as described below, the value vector including a set of the temporal change rates of the concentrations of the nutrient components, of the concentrations of the products, and of the number density of the cells, and the intracellular reaction rate, on a screen 20, for example, as shown in Fig. 2. The output display device 9 also displays the predicted value and actual value of the concentrations of nutrient components, the concentrations of the products, and the number density of the cells on the screen 20, for example, as shown in Fig. 3.

The storage device 8 which is configured to store a program used to perform a calculation in the arithmetic processing unit 7. The arithmetic processing unit 7 performs the following calculations in accordance with the program.

The measuring device 4 measures the concentrations of the nutrient components such as sugar typified by glucose and amino acids typified by glutamine, the concentrations of the products such as the concentration of lactic acid, the concentration of ammonia, and the concentrations of protein, oxygen concentration, and the number density of the cells. At the first stage, the difference in the measured concentration between two measurement times is divided by the time period $\Delta t$ and the number density of the cells $X_a$ to calculate a consumption rate or formation rate per unit of time and per unit of the number of cells.

For example, the concentration of glutamine Gln is calculated using Equation 1. Here, a subscript obs represents an observed value. Also, Gln represents the concentration of glutamine with a unit of mg/L. $X_a$ represents the number density of the cells with a unit of cells/mL.

[Equation 1]

$$\frac{1}{Xa}\frac{dGln}{dt} = \frac{Gln^{obs}(t+\Delta t) - Gln^{obs}(t)}{Xa^{obs}(t) \cdot \Delta t} \qquad \cdots (1)$$

The number density of the cells is calculated using Equation 2. $\mu$ is called a specific growth rate, and is used with a unit of 1/s.

[Equation 2]

$$\mu = \frac{Xa^{obs}(t+\Delta t) - Xa^{obs}(t)}{Xa^{obs}(t) \cdot \Delta t} \qquad \cdots (2)$$

Intracellular conditions are difficult to completely identify. An intracellular occurrence, at a chemical reaction level, is a collective of a plurality of chemical reactions which are accompanied by a material conversion including the intake of the substrate component, and the external secretion of the products through a large number of metabolic intermediates. It is difficult to pick up all reactions which take place in the cells. However, it is possible to collect a relatively small number of the main reactions to create a mathematical model for an intracellular chemical reaction system. The mathematical model which describes the intracellular reaction system is constructed using the intracellular reaction rate as an internal condition variable which describes the intracellular conditions. The intracellular reaction rate is determined by solving this model.

The circuit network of the intracellular reaction rate is stored in the storage device 8. Specifically, material names and connections between arrows corresponding two of the material name indicated by F1 to F30 indicating chemical reactions of the circuit network shown in Fig. 2 are stored. In Fig. 2, Glutamine and Lactate and the like represent the concentrations of the nutrient components and products in the culture medium outside the cells. Pyruvate and Oxaloacetate and the like represent the concentrations of the intracellular metabolic intermediates. Each of the arrows F1 to F30 respectively represents one chemical reaction. For example, F1 represents a chemical reaction in which Glucose is converted to Pyruvate. It is also shown that the reaction rate thereof is designated by F1.

Next, the equation derived from the circuit network of the intracellular reaction rates is then described. The vectors formed by aligning reaction rates of these reactions can be considered as an internal condition variable which describes the cells. Particularly, under conditions in which the culture environment will not be drastically changed, the intracellular metabolic reaction is in an approximately steady state. Therefore, the concentration of each metabolic intermediate is constant, thus resulting in zero of a total sum of the reaction rates related to the formation and consumption of each metabolic intermediate. It is possible to determine all reaction rates which are included in the mathematical model for the intracellular reaction rate system if the above condition, the inflow rate of the substrate and the exit rates of the products, are used.

For example, the concentration of metabolic intermediate Pyruvate shown in Fig. 2 is constant under the assumption of a steady state. Thus, the total sum of the reaction rates at which the metabolic intermediate enters or exits Pyruvate is zero. As can be seen in Fig. 2, Equation 3 is established.

[Equation 3]

$$F1 - F2 - F4 + F10 - F13 = 0 \qquad \cdots (3)$$

Thus, the same number M of equations as that of the metabolic intermediates included in the circuit network shown in Fig. 2 are derived. Also, for example, the reaction rate of a reaction F6 in which Glutamine is converted to Glutamate as shown in Fig. 2 is calculated from Equation 1 using the measured value of the concentration of Glutamine, thus resulting in the establishment of Equation 4.

[Equation 4]

$$F6 = \frac{Gln^{obs}(t + \Delta t) - Gln^{obs}(t)}{Xa^{obs}(t) \cdot \Delta t} \qquad \cdots (4)$$

As shown above, the temporal change rates of the concentrations of the components contained in the culture medium is dealt with as input data in the equation system. The same number N of this relational equation as that of the concentrations of the components contained in the culture medium being measured are given. The equations which have thus been derived establish a linear simultaneous equation including a total number of equations of M+N included therein.

If the total number M+N of the equations included in the linear simultaneous equation is equal to the number of the reactions considered in the model, for example, to 30 in the example shown in Fig. 2, the linear simultaneous equation can be solved, thereby all reaction rates being able to be determined. Thus, the determined values are shown above the corresponding arrows in Fig. 2. Also, the determined reaction rates are indicated on the time axis as shown in Fig. 3. Thus, all reaction rates are determined. Therefore, it is possible to estimate what result is obtained based on how the control is performed, and thereby a target can be selected to perform a control in consideration of the intracellular reaction rate.

If the total number M+N of equations included in the linear simultaneous equation exceeds the number of the reaction rate considered in the model, several measured temporal change rates of the concentrations of the components contained in the culture medium are not necessary to be used as input data, and can be used as verification data.

On the contrary, if the total number M+N of equations included in the linear simultaneous equation is less than the number of the reaction rate considered in the model, a solution cannot be uniquely determined. In this case, the set of reaction rates that meets target conditions can be determined by further simplifying the model to reduce the number of reaction rates to be considered, or by specifying a target function such as, for example, the minimization of the formation reaction rate F30 of Ammonia to make a change to a linear programming problem.

Then, the intracellular reaction rate determined in the manner described above is expressed as the function of the concentrations of the nutrient components and products contained in the culture medium. For example, a reaction rate F1 that provides the conversion of Glucose to Pyruvate is expressed by F1=F1(Glc, Gln, Lac, Amm) as a function of the concentrations of Glucose (Glc), Glutamine (Gln), Lactic acid (Lac), and Ammonia (Amm) contained in the culture medium. When the function is determined explicitly, an intracellular reaction rate desired for control can be controlled by adjusting the concentrations of the components contained in the culture medium such as Glc, Gln, Lac, and Amm.

As described above, the intracellular reaction rate can be determined from the equation of the circuit network using, as an input data, the temporal change rates of the concentrations of the nutrient components, of the concentrations of the products, and of the number density of the cells. Therefore, if the temporal change rates of the concentrations of the nutrient components, of the concentrations of the products, and of the number density of the cells are provided as the functions of the concentrations of the nutrient components, and of the concentrations of the products, the function F1 is determined explicitly. Then, these functions are estimated using the actual measurement value obtained by the measuring device.

That is, the model estimation equations for the temporal change rates of the concentrations of the nutrient components, of the concentrations of the products, and of the number density of the cells are established as shown in Equations 5 to 15.

[Equation 5]

$$\frac{dx_a}{dt} = \mu X_a - k_d X_a \qquad \cdots (5)$$

[Equation 6]

$$\frac{dGlc}{dt} = -q_{Glc} X_a \qquad \cdots (6)$$

[Equation 7]

$$\frac{dGln}{dt} = -q_{Gln} X_a - \kappa_{Gln} Gln \qquad \cdots (7)$$

[Equation 8]

$$\frac{dLac}{dt} = q_{Lac} X_a - \kappa_{Lac} Lac \qquad \cdots (8)$$

[Equation 9]

$$\frac{dAmm}{dt} = q_{Amm}X_a + \kappa_{Gln}Gln \qquad \cdots (9)$$

[Equation 10]

$$\mu = \mu_{max}\frac{Glc}{Glc+K_{Glc}} \cdot \frac{Gln}{Gln+K_{Gln}} \cdot \frac{1}{\left[1+\dfrac{Lac^2}{K_{Lac}}\right]} \cdot \frac{1}{\left[1+\dfrac{Amm^2}{K_{Amm}}\right]} \qquad \cdots (10)$$

[Equation 11]

$$k_d = k_{d0}e^{-a\mu} \qquad \cdots (11)$$

[Equation 12]

$$q_{Glc} = \frac{\mu}{Y_{Glc}} + m_{Glc} + q_{E,Glc}^{Glc}\frac{Glc-Glc_0^{Glc}}{\left(Glc-Glc_0^{Glc}\right)+K_{Glc}^{Glc}} + q_{E,Glc}^{Gln}\frac{Gln-Gln_0^{Glc}}{\left(Gln-Gln_0^{Glc}\right)+K_{Gln}^{Glc}}$$

$$\cdots (12)$$

[Equation 13]

$$q_{Gln} = \frac{\mu}{Y_{Gln}} + m_{Gln} + q_{E,Gln}^{Gln}\frac{Gln-Gln_0^{Gln}}{\left(Gln-Gln_0^{Gln}\right)+K_{Gln}^{Gln}} \qquad \cdots (13)$$

[Equation 14]

$$q_{Lac} = \frac{\mu}{Y_{Lac}} + m_{Lac} + q_{E,Lac}^{Glc} \frac{Glc}{Glc + K_{Glc}^{Lac}} \qquad \cdots (14)$$

[Equation 15]

$$q_{Amm} = \frac{\mu}{Y_{Amm}} + m_{Amm} + q_{E,Amm}^{Glc} \frac{Glc}{Glc + K_{Glc}^{Amm}} + q_{E,Amm}^{Gln} \frac{Gln}{Gln + K_{Gln}^{Amm}} \qquad \cdots (15)$$

In the above equations, $K_d$ is a death rate represented by a unit of 1/s. Amm is the concentration of Ammonia represented by a unit of mg/L. Lac is the concentration of lactic acid represented by a unit of mg/L. Glc is the concentration of glucose represented by a unit of mg/L. $\mu_{max}$, $\kappa_{d0}$, $\alpha$, $K_i$, $K_j^i$, $Y_i$, $m_i$, $q_{Ei}^j$, $\kappa_j$ (i, j =Glc, Gln, Lac, Amm) are the model parameters used in the model estimation equation. The best estimation equation is determined by fitting the model parameters by using a method of least squares using the actual measurement values of the concentrations of the nutrient components, concentrations of the products, and number density of the cells, and using the temporal change rates thereof calculated by using the above described algorithm.

A given intracellular reaction rate Fi can be determined as the functions of the concentrations of the nutrient components and concentrations of the products such as Fi = Fi(Glc, Gln, Lac, Amm) by solving the equation of the circuit network using the best estimation equation as the input data.

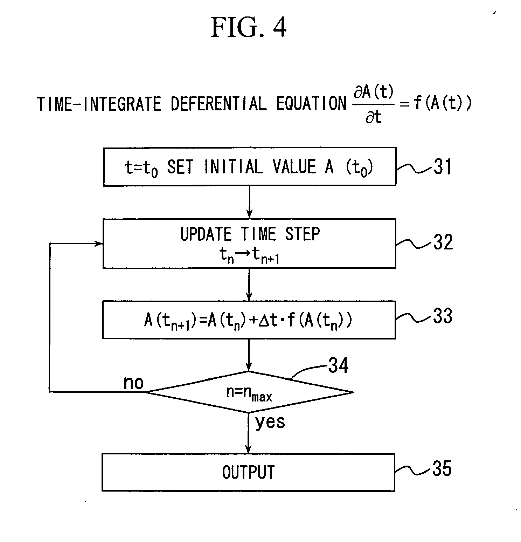

The concentrations of the components contained in the culture medium can be estimated by means of the determined model estimation equation. As a result, the concentrations of the nutrient components, the concentrations of the products, and the number density of the cells in the chronological future can be calculated by numerically integrating the model estimation equation of the temporal change rates of the concentrations of the nutrient components, of the concentrations of the products, and of the number density of the cells by means of the algorithm shown in Fig. 4. Thus, the results can be displayed as shown in Fig. 3.

In the numerical integration, as shown in Fig. 4, the initial value of the function to be integrated is set at step 31. At step 32, a time step is increased by 1 to be updated. At step 33, the difference of the function is calculated. At step 34, it is judged whether or not the number n of times the time step has been updated reaches nmax. If n does not reach nmax, the process is returned to step 32 to sequentially repeat steps 33 and 34. When the number of times the time step has been updated reaches nmax, output is performed at step 35.

The predictive control of the culture tank is performed using such calculation results. For example, in the case where a control is performed so as to maximize the concentrations of the products, a target function of maximizing the concentrations of the products can be specified to determine the set of the reaction rates that meets the target conditions. In this case, the number density of the cells can be determined as a target function. A dynamic control target value to be controlled, such as the concentrations of the nutrient components, and the concentrations of the products, can be determined by solving the circuit network equation using the above described best estimation equation as the input data. Therefore, based on the state of the culture medium measured by the sampler 15, and the measured values of the concentrations of the products, concentration of oxygen, and number density of the cells measured by the measuring device 4, the controller 6 controls the nutrient component supply device 5, the valve 11, and the driving device 10 to control the supply of nutrient components such as sugar and amino acid, the concentrations of oxygen gas and carbon dioxide, and stirring. These results of control are displayed on the screens shown in Figs. 2 and 3 to visually show whether the control is normally performed.

According to the present embodiment, the operation control of the culture tank can suitably be performed using the observation variables in the culture medium such as the concentrations of the nutrient components, the concentrations of the products, and the number density of the cells, and the intracellular reaction rate as the state variables of the culture tank.

**Claims**

1. An operation controller of a culture tank comprising:

   a culture tank (1) in which a culture medium for culturing animal cells or a microorganism is enclosed;
   a measuring device (4, 14) for measuring concentrations of nutrient components, concentrations of products, and the number density of cells in the culture medium;
   a supply device (5) for replenishing the nutrient components and oxygen into the culture medium; and
   an arithmetic processing unit (7) in which the measured values from the measuring device are inputted, and which thus controls the supply device, wherein
   the arithmetic processing unit solves an equation derived from a circuit network of intracellular reaction rates stored in a storage device (8) by using temporal rates of change of the concentrations of the nutrient components, of the concentrations of the products, and of the number density of the cells as input data, such that the arithmetic processing unit calculates a desired intracellular reaction rate, and
   the arithmetic processing unit thus controls the supply device depending on the result of the calculation to control the concentrations of the components contained in the culture medium.

2. An operation controller of a culture tank comprising:

   a culture tank (1) in which a culture medium for culturing animal cells or microorganism is enclosed;
   a measuring device (4, 14) for measuring concentrations of nutrient components, concentrations of products, and the number density of the cells in the medium;
   a supply device (5) for replenishing the nutrient components and oxygen into the culture medium; and
   an arithmetic processing unit (7) in which the measured values from the measuring device are inputted, and which thus controls the supply device, wherein
   the arithmetic processing unit specifies target conditions such as a formation reaction rate and the like, and
   the arithmetic processing unit solves an equation derived from a circuit network of intracellular reaction rates stored in a storage device (8) by using temporal rates of change of the concentrations of the nutrient components, of the concentrations of the products, and of the number density of the cells as input data, such that the arithmetic processing unit calculates a set of intracellular reaction rates which meets the target conditions, and the arithmetic processing unit thus controls the supply device depending on the result of the calculation to control the concentrations of the components contained in the culture medium.

3. The operation controller of a culture tank according to claim 1 or 2, wherein the circuit network of intracellular reaction rates is a construction of a mathematical model which describes the intracellular reaction rates by using the intracellular reaction rates as an internal state variables of the cells.

4. The operational controller of a culture tank according to any of claims 1 to 3, wherein material names and connections between corresponding two of the material names shown by arrows indicating chemical reactions in the circuit network are stored in the storage device.

5. The operation controller of a culture tank according to any of claims 1 to 4, comprising an output display device (9), wherein the temporal rates of change of the concentrations of the nutrient components, of the concentrations of the products, and of the number density of the cells, and value vectors each including the set of the intracellular reaction rates are displayed on a screen thereof.

6. The operation controller of a culture tank according to any of claims 1 to 5, wherein the arithmetic processing unit is configured to store a model estimation equation which is described as a function of the concentrations of the nutrient components, the concentrations of the products, and the number density of the cells, and the arithmetic processing unit is configured to estimate the temporal change rates of the concentrations of the nutrient components, of the concentrations of the products, and of the number density of the cells by fitting model parameters in the model estimation equation based on the measured values.

7. The operation controller of a culture tank according to claim 6, wherein a time integration is applied to the model estimation equation which provides the temporal change rates of the concentrations of the nutrient components, of the concentrations of the products, and of the number density of the cells, the concentrations of the nutrient components, the concentrations of the products, and the number density of the cells in the chronological future are estimated, and thus the estimated values and actually measured values are displayed on the screen of the output display device.

EP 1 816 188 A1

# FIG. 1

O₂ VENTILATION ON LIQUID SURFACE

NUTRIENT COMPONENT SUPPLY DEVICE — 5

10

13

DISCHARGED O₂ AND CO₂

2a
11a

β

14

GAS CHROMATOGRAPHY

3

1

SAMPLER

MEASURING DEVICE

ARITHMETIC PROCESSING UNIT — 7

STORAGE DEVICE — 8

15

4

O₂ VENTILATION IN LIQUID

MEASURED VALUE

OUTPUT DISPLAY DEVICE — 9

11b   2b

CONTROLLER

CONTROL TARGET VALUE

6

FIG. 2

~20

EP 1 816 188 A1

Cell Line: [_____]　　　Fermenter: [_____]　　　Start Date: [2005/08/22 15:58]

Consumption
☐ Glc ☐
☐ Gln ☐
☐ Ala ☐
☐ Glu ☐
☐ Pro ☐
☐ Asp ☐
☐ Asn ☐
☐ Ser ☐
☐ Gly ☐
☐ Lys ☐
☐ Ile ☐
☐ Leu ☐
☐ Thr ☐
☐ Val ☐

Carbohydrates　　Ribose-5-P
　　　　　　　　　　F17
　　　F18
Lipids ←　　　Glucose　　→ F11 →　Serine　　→ F12 →　Glycine
　　　F19
NADPH　F20　　　　　　Lysine　　Isoleucine　　　Leucine
　　　　　　　　　　　　　　F14　　F15　　　　F16
Alanine ←　F4　Pyruvate　　→ F13 →　Acetyl-CoA
$(X7,,Y7)$　　　　$(X7,,Y7)$
　　　F2　　　　F1
　　　　　　　F10　　　$(X6,,Y6)$　　　　　F3
Lactate
$(X7,,Y7)$　　　　　　　　　F3　　　　　　　$(X8,,Y8)$
　　$(X5,$　Oxaloacetate　$(X6,,Y6)$　　　　　Coa
　　$Y5)$　　　　　　　　　　　Citrate　　Glutamine
　　　　　F8　　F27　　　　　　　　　　　F6
　　　　　　　→ ATP　　　　　F3　　Glutamate　- - -→ NH3
　　　　　　F26　　　　　　$(X1,,Y1)$　F5　　　F30
Aspartate　　TCA CyCle　α-Ketoglutarate
　F9　　　$(X4,,Y4)$　　　　　F23　　F7
Asparagine　　　　　　　　　$(X2,,Y2)$　　Proline
　　　　　　CO2　　　O2　Succiny-CoA ←　F21 ←　Threonine
　　　　　F29　　　F28　　　　　　　　　　F22
DNA　Fumarate　　　　　　　　　F15　　　Valine
RNA ←　　　　　F24　　　Isoleucine
　　F25
Aspartate　　　$(X3,,Y3)$

Production
☐ Lac ☐
☐ NH3 ☐
☐ Amb ☐

RQ
☐ OUR ☐
☐ CER ☐

(P/O)
☐ N ☐

# FIG. 3

# FIG. 4

TIME-INTEGRATE DEFERENTIAL EQUATION $\dfrac{\partial A(t)}{\partial t} = f(A(t))$

$t=t_0$ SET INITIAL VALUE $A(t_0)$ — 31

UPDATE TIME STEP
$t_n \rightarrow t_{n+1}$ — 32

$A(t_{n+1}) = A(t_n) + \Delta t \cdot f(A(t_n))$ — 33

34

no    $n = n_{max}$

yes

OUTPUT — 35

**EP 1 816 188 A1**

**European Patent Office**

**EUROPEAN SEARCH REPORT**

Application Number

EP 06 01 8900

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 1 270 718 A (JAPAN TISSUE ENGINEERING CO LT [JP]; TAYA MASAHITO [JP]) 2 January 2003 (2003-01-02) * claim 1 * | 1 | INV. C12M1/36 |
| A | WO 00/70014 A1 (LARSEN EBBE BUSCH [DK]) 23 November 2000 (2000-11-23) * page 9, line 23 - line 28 * | 1 | |
| A,D | JP 05 103663 A (TOYO BOSEKI) 27 April 1993 (1993-04-27) * abstract * | 1 | |
| A,D | JP 2002 095496 A (TAKASAGO THERMAL ENGINEERING) 2 April 2002 (2002-04-02) * abstract * | 1 | |
| A,D | JP 09 065873 A (ORIENTAL YEAST CO LTD) 11 March 1997 (1997-03-11) * abstract * | 1 | |
| A | JP 57 144978 A (HITACHI LTD) 7 September 1982 (1982-09-07) * abstract * | 1 | TECHNICAL FIELDS SEARCHED (IPC) C12M |

The present search report has been drawn up for all claims

| Place of search | Date of completion of the search | Examiner |
|---|---|---|
| Berlin | 8 May 2007 | Clement, Jean-Paul |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

14

## ANNEX TO THE EUROPEAN SEARCH REPORT
## ON EUROPEAN PATENT APPLICATION NO.

EP 06 01 8900

08-05-2007

| Patent document cited in search report | | Publication date | Patent family member(s) | | Publication date |
|---|---|---|---|---|---|
| EP 1270718 | A | 02-01-2003 | AU | 4462501 A | 15-10-2001 |
| | | | WO | 0175070 A1 | 11-10-2001 |
| | | | JP | 2001275659 A | 09-10-2001 |
| | | | US | 2003054335 A1 | 20-03-2003 |
| WO 0070014 | A1 | 23-11-2000 | AT | 356196 T | 15-03-2007 |
| | | | AU | 4744100 A | 05-12-2000 |
| | | | CA | 2373288 A1 | 23-11-2000 |
| | | | EP | 1183326 A1 | 06-03-2002 |
| | | | MX | PA01011873 A | 04-09-2003 |
| | | | US | 6492135 B1 | 10-12-2002 |
| JP 5103663 | A | 27-04-1993 | NONE | | |
| JP 2002095496 | A | 02-04-2002 | NONE | | |
| JP 9065873 | A | 11-03-1997 | NONE | | |
| JP 57144978 | A | 07-09-1982 | JP | 1291057 C | 29-11-1985 |
| | | | JP | 60015306 B | 18-04-1985 |

EPO FORM P0459

**EP 1 816 188 A1**

## REFERENCES CITED IN THE DESCRIPTION

### Patent documents cited in the description

- JP 5103663 A
- JP 2002095496 A
- JP 9065873 A